# EUROPÄISCHE PATENTSCHRIFT

(11) **EP 0 932 360 B1**
(45) Veröffentlichungstag und Bekanntmachung des Hinweises auf die Patenterteilung: **12.06.2002**
(21) Anmeldenummer: 97944841.2
(22) Anmeldetag: 06.09.1997
(51) Int. Cl.: A47L 13/16, C08L 1/06, C08J 9/00

(54) **BIOCID AUSGERÜSTETES, FASERVERSTÄRKTES SCHWAMMTUCH**
BIOCIDE IMPREGNATED FIBRE REINFORCED SPONGE MATERIAL
TISSU EPONGE RENFORCE PAR DES FIBRES, IMPREGNE DE BIOCIDE

(30) Priorität: 18.10.1996 DE 29618058 U
(43) Veröffentlichungstag der Anmeldung: 04.08.1999
(73) Patentinhaber: Kalle Nalo GmbH & Co. KG, 65203 Wiesbaden (DE)
(72) Erfinder: MANS, Leo, D-55120 Mainz (DE); HAMMER, Klaus-Dieter, D-55120 Mainz (DE)
(74) Vertreter: Zounek, Nikolai, Dipl.-Ing.
(86) Internationale Anmeldenummer: EP9704849
(87) Internationale Veröffentlichungsnummer: WO9817165

(56) Entgegenhaltungen:
- DE-A- 2 834 914
- DE-A- 2 841 749
- DE-B- 1 297 852
- GB-A- 887 430
- US-A- 4 940 631
- US-A- 5 441 742
- PATENT ABSTRACTS OF JAPAN vol. 14, no. 423 (C-757), 12.September 1990 & JP 02 160843 A (LION CORP), 20.Juni 1990, & DATABASE WPI Week 9031 Derwent Publications Ltd., London, GB; AN 234392
- PATENT ABSTRACTS OF JAPAN vol. 14, no. 404 (M-1018), 31.August 1990 & JP 02 153723 A (LION CORP), 13.Juni 1990, & DATABASE WPI Week 9030 Derwent Publications Ltd., London, GB; AN 226787

## Beschreibung

Die vorliegende Erfindung betrifft ein nach dem Viskoseverfahren hergestelltes, faserverstärktes Schwammtuch, das sich als vielseitig einsetzbares Putz- und Reinigungstuch, insbesondere für den privaten Haushalt, eignet.

Die Schwammtuchherstellung nach dem Viskoseverfahren ist seit langem bekannt. Darin wird zunächst Zellstoff, insbesondere Holzzellstoff, mit Natriumhydroxid und Schwefelkohlenstoff in eine alkalische Cellulosexanthogenatlösung, die sogenannte Viskoselösung, übergeführt. Parallel dazu werden Baumwollkämmlinge mit verdünnter, detergentienhaltiger NaOH entfettet und mercerisiert. Viskoselösung und Baumwollfasern werden dann zu einer homogenen Masse vermischt, allgemein mit Hilfe eines Kneters. Anschließend wird Glaubersalz (Natriumsulfat-Decahydrat) hinzugefügt und ebenfalls gleichmäßig vermischt. Diese Schwammtuch-Rohmasse wird dann auf eine gelochtes Endlosband in der jeweils gewünschten Höhe aufgetragen. Die Regenerierung der Cellulose erfolgt dann in einem erwärmten, alkalischen Koagulationsbad. Sie kann auch in einem sauren Medium, beispielsweise verdünnter Schwefelsäure, durchgeführt werden. Dabei wird die innere Verstärkung in den Schwammtuchkörper eingebunden.

Das Glaubersalz weist einen sehr niedrigen Schmelzpunkt (etwa 32 bis 33 °C) auf. Es wird daher in dem Koagulationsbad aufgeschmolzen und herausgelöst. An Stelle der Salzkristalle bleiben Poren und Hohlräume zurück. Schließlich wird das Schwammtuch ausgewaschen, um es von Salzresten und anhaftenden Reaktionsprodukten zu befreien. Nach dem Trocknen wird es in schmale Bahnen geschnitten, die wiederum aufgerollt werden. Die Rollenware kann dann zu Tüchern der gewünschten Größe konfektioniert werden. Falls gewünscht, kann sie vorher noch bedruckt werden.

Faserverstärkte Schwammtücher sind im trockenen Zustand jedoch spröde. Sie werden daher nur in wenigen Ländern in dieser Form in den Handel gebracht. Befeuchtete Schwammtücher sind demgegenüber flexibler und haben einen wesentlichen besseren "Griff'. Um das Schwammtuch feucht zu halten, werden die Schwammtuchbahnen mit der Lösung eines hygroskopischen Salzes, insbesondere Magnesiumchlorid, imprägniert. Überschüssige Flüssigkeit wird mit Hilfe eines Quetschwalzenpaares entfernt. Die feuchten Tücher werden dann verpackt, normalerweise in einer Kunststoffolie. Dabei können sich in der Verpackung jedoch Mikroorganismen, insbesondere Bakterien und Pilze, vermehren, was sich in Form von dunklen Flecken auf den Schwammtüchern äußert.

Es stellte sich daher die Aufgabe, diese Mikroorganismen abzutöten oder zumindest an der Ausbreitung zu hindern.

Gemäß der vorliegenden Erfindung wird die Aufgabe gelöst durch ein Schwammtuch auf der Basis von regenerierter Cellulose, das mit einer Innenverstärkung versehen ist und und dadurch gekennzeichnet ist, daß die Fällung und Regenerierung in einem aus Glaubersalz, NaOH und Wasser bestehenden Bad mit einem pH-Wert von 13 oder mehr erfolgt und daß die Innenverstärkung aus Viskosefasern mit einer Stapelfaserlänge von 5 bis 50 mm besteht und daß es mit einem biocid wirkenden Mittel imprägniert ist, das nach der Regeneration aufgebracht wird. Die Viskosefasern sind im Vergleich zu den bisher üblichen Baumwollfasern in einer wesentlich gleichmäßigeren Qualität erhältlich. Bei der Entkörnung der Baumwollfasern werden zudem nicht alle Samenkörner und Bestandteile der Baumwollkapsel entfernt. Diese Verunreinigung macht sich besonders bei ungefärbten oder in hellen Farben eingefärbten Schwammtüchern in unerwünschter Weise bemerkbar. Die Samenkörner sind darin als kleine schwarze Punkte sichtbar. Die Samenkörner lassen sich zwar entfernen, doch ist das mit einem relativ großen Aufwand verbunden. Die Farbe der natürlichen Baumwollfasern ist zudem Schwankungen unterworfen. Schließlich haben Baumwollfasern den Nachteil, daß sie vorher entfettet werden müssen. Reste der zur Entfettung verwendeten Tenside führen leicht zu Störungen, insbesondere durch Schaumbildung, bei der Herstellung des Schwammtuches. Bekannt war ferner, daß biocid wirksame quaternäre Ammoniumsalze, speziell Benzalkonium-chloride, ihre Wirksamkeit in Gegenwart von Baumwollfasern verlieren (Martindale, The Extra Pharmacopoeia, 28th ed., London [1982], Seite 549).

Bevorzugte biocid wirksame Mittel sind dabei Isothiazolon-, Benzisothiazolon- und Benzimidazol-Derivate, insbesondere solche der folgenden Formeln 1 bis 12.

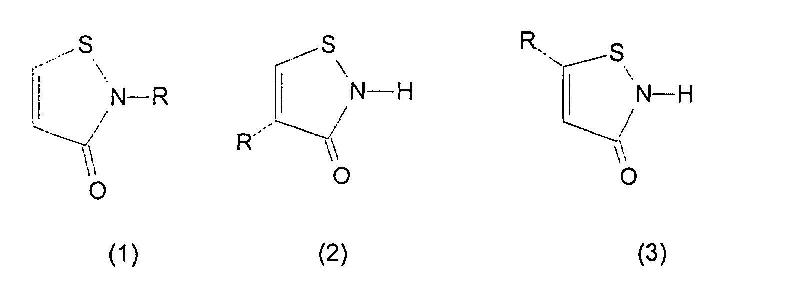

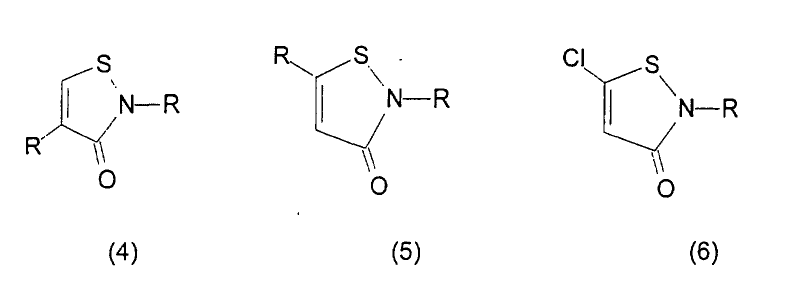

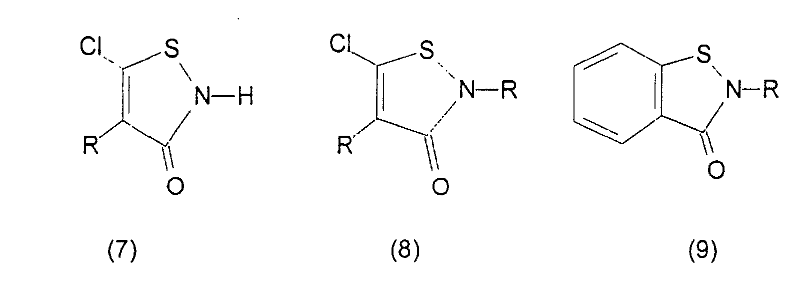

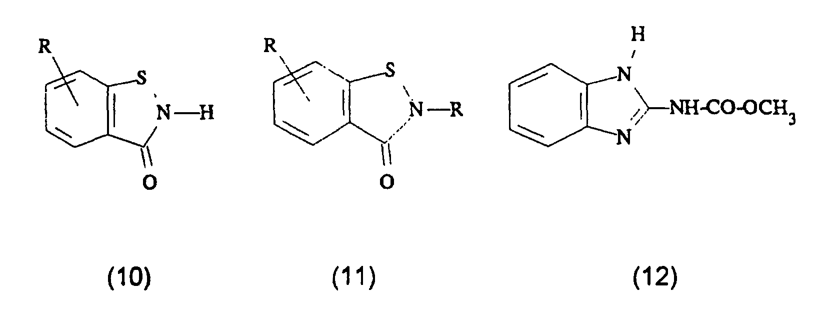

Der Rest R steht in den vorgenannten Formeln für einen Alkyl-, Alkenyl- oder Alkadienylrest, der im allgemeinen 1 bis 20 Kohlenstoffatome umfaßt und vorzugsweise geradkettig ist. Von diesen sind die geradkettigen Alkylreste mit 6 bis 18 Kohlenstoffatomen besonders bevorzugt. Als größter praktikabler N-Alkylrest hat sich der Stearylrest erwiesen. Verbindungen, deren N-Alkylrest 14 Kohlenstoffatome oder weniger umfaßt, zeigen allgemein eine höhere baktericide Wirkung. Mit abnehmender Kettenlänge steigt jedoch auch die Wasserlöslichkeit. Ist mehr als eine Alkylgruppe vorhanden, dann beträgt die Gesamtzahl der darin enthaltenen Kohlenstoffatome vorzugsweise ebenfalls nicht mehr als 20. In der Verbindung der Formel 7 kann der Rest R darüber hinaus auch für ein Chloratom stehen. Bei den chlorsubstituierten Isothiazolonen, d.h. bei den Verbindungen 6 bis 8, umfaßt die Alkylgruppe allgemein nur 1 bis 12, bevorzugt sogar nur 1 bis 8, Kohlenstoffatome. Durch den zweiten Chlorsubstituenten wird die bakterizide Wirkung der Verbindung verstärkt, zudem ihre Wasserlöslichkeit verringert. In den Verbindungen der Formeln 9, 10 und 11 ist der Alkylrest allgemein kürzer. Er enthält nur 1 bis 4 Kohlenstoffatome, wobei Methyl-, Ethyl- und Propylreste wiederum bevorzugt sind.

Daneben kann R auch für einen Cycloalkylrest stehen, der im allgemeinen 3 bis 12 Kohlenstoffatome umfaßt. In den chlorsubstituierten Isothiazolonverbindungen der Formeln 6 bis 8 umfaßt der Cycloalkylrest allgemein nur 3 bis 8 Kohlenstoffatome, wobei der Cyclohexylrest besonders hervorgehoben ist. Besonders bevorzugt sind die Verbindungen der Formeln 1, 3, 6 und 9. Die Verbindung der Formel 12 (2-Methoxycarbonylamino-benzimidazol) ist auch unter dem INN-Namen Carbendazim bekannt. Weiterhin sind auch 1-Butylcarbamoyl-2-methoxycarbonylamino-benzimidazol (Benomyl), 2-Thiazol-4-yl-benzimidazol (Thiabendazol), und 2-Furan-2-yl-benzimidazol (Fuberidazol) geeignet.

Fungicid und/oder baktericid wirken auch quaternäre Ammoniumsalze mit langkettigen, in der Regel gesättigten, aber auch ungesättigten Alkylgruppen mit 6 bis 24, bevorzugt 10 bis 18 Kohlenstoffatomen. Von diesen kationischen, tensioaktiven Ammoniumsalzen besonders zu nennen sind Di-(C₁₀-C₁₈)alkyldimethylammoniumchloride (wie Didecyl-dimethyl-ammoniumchlorid), (C₈-C₁₈)Alkyl-trimethyl-ammoniumchloride (wie Trimethyl-octyl-ammoniumchlorid, Decyl-trimethyl-ammoniumchlorid und Hexadecyl-trimethylammoniumchlorid), Sojaalkyl-trimethyl-ammoniumchlorid, Dicocosalkyl-dimethyl-ammoniumchlorid, Alkyl-benzyl-dimethyl-ammoniumchloride (wie Benzyl-dimethyl-stearyl-ammoniumchlorid, Benzyl-cocosalkyl-dimethyl-ammoniumchlorid oder Cocosalkyl-(2,4-dichlor-benzyl)-dimethyl-ammoniumchlorid. Häufig werden auch Gemische von quaternären Ammoniumsalzen verschieden langer Fettsäuren eingesetzt (Cocosalkyl und Sojaalkyl steht für ein Gemisch verschieden langer, gesättigter und ungesättigter Alkylgruppen). Das Anion in den Ammoniumsalzen muß nicht notwendig Chlorid sein. Ebenso gut kann das Anion auch Bromid, Acetat, Propionat, Sorbat, Benzoat oder Sulfat sein. Diese Verbindungen sind beispielsweise in der EP-A 286 009 beschrieben.

Gegenstand der vorliegenden Erfindung ist schließlich auch ein Schwammtuch auf der Basis von regenerierter Cellulose, das mit einer Innenverstärkung versehen ist, dadurch gekennzeichnet, daß die Fällung und Regenerierung in einem aus Glaubersalz, NaOH und Wasser bestehenden Bad mit einem pH-Wert von 13 oder mehr erfolgt und daß die Innenverstärkung aus Baumwollfasern mit einer Stapelfaserlänge von 5 bis 50 mm besteht und daß es mit einem baktericid wirkenden Mittel imprägniert ist, das nach der Regeneration aufgebracht wird.

Das baktericid wirkende Mittel ist darin vorzugsweise eines der bereits genannten Isothiazolon-, Benzisothiazolon- oder Benzimidazol-Derivate.

Als baktericide Mittel in dem erfindungsgemäßen Schwammtuch können auch Dipyridyldisulfid und dessen Bis-N-oxid eingesetzt werden, speziell Dipyridin-2-yl-disulfid. Ferner können auch die ebenfalls in der EP-A 286 009 beschriebenen 1-Alkyl- und 1-Alkenyl-pyridiniumsalze (wie 1-Lauryl-pyridiniumchlorid) eingesetzt werden. Verwendbar sind auch Biguanidverbindungen, wie sie in der US-A 4 675 347 beschrieben sind. Weiterhin geeignet sind auch Penicilline, Cephalosporine, Tetracycline und andere antibakteriell wirksame Verbindungen, soweit für diesen Zweck amtlich zugelassen. Schließlich können auch mehrere der vorgenannten Mittel miteinander kombiniert sein.

Die biocide Ausrüstung kann auch durch Behandeln mit Glycerin oder Propan-1,2-diol (Propylenglykol) bewirkt werden. Um wirksam zu sein, müssen sie in dem Schwammtuch in einer Menge vorhanden sein, die ausreicht, um die Wasseraktivität (den sogenannten a_{w}-Wert) auf 0,9 oder weniger herabzusetzen. Bei einem a_{w}-Wert von 0,85 und weniger wird auch die Entwicklung von Schimmelpilzen gehemmt (siehe E. Lück, Z. Lebensm. Unters.-Forsch. 153 [1973] 42 - 52). Viele der genannten Verbindungen wirken nicht nur gegen Pilze und Bakterien, sondern auch gegen Hefen, Algen und andere Mikroorganismen.

Der Gewichtsanteil der biocid wirksamen Verbindung(en) ist von der Art der Mikroorgansmen und der Konstitution der jeweiligen Verbindung abhängig. Im Falle eines (C₁₂-C₁₈)Alkyl-benzyl-dimethyl-ammoniumchlorids liegt die fungicid wirksame Menge bei etwa 1100 bis 1600 ppm, bezogen auf das Trockengewicht des Schwammtuchs. Beim Didecyl-dimethyl-ammoniumchlorid reicht bereits 1 ppm aus, um eine algicide Wirkung zu erreichen.

Das biocid wirkende Mittel kann dem erwähnten Befeuchtungsbad hinzugefügt sein. Das Bad enthält bevorzugt eine etwa 2 bis 8 gew.-%ige, insbesondere eine etwa 4 gew.-%ige Magnesiumchloridlösung. Dieses Herstellungsverfahren ist apparativ besonders günstig. Es kann aber auch in ein separates Behandlungsbad gegeben werden. Die befeuchteten Schwammtücher enthalten etwa 0,01 bis 0,4 Gew.-%, bevorzugt etwa 0,03 bis 0,25 Gew.-% an MgCl₂, jeweils bezogen auf das Trockengewicht des Schwammtuches. Die Imprägnierung mit dem hygroskopischen Salz und die Imprägnierung mit der biocid wirkenden Verbindung sind prinzipiell jedoch unabhängig voneinander. Auch nichtbefeuchtete Schwammtücher können somit antibakteriell ausgerüstet sein.

Die als Innenverstärkung dienenden Baumwoll- oder Viskosefasern in dem erfindungsgemäßen Schwammtuch haben bevorzugt eine Stapelfaserlänge von 10 bis 30 mm. Die Baumwollfasern werden wie eingangs beschrieben vor ihrer Verwendung entfettet und gegebenenfalls mercerisiert. Der Anteil der Innenverstärkung beträgt allgemein etwa 5 bis 50 Gew.-%, bevorzugt 10 bis 40 Gew.-%, jeweils bezogen auf das Trockengewicht des Schwammtuches.

Die weitere Herstellung erfolgt dann vorzugsweise, wie eingangs beschrieben, durch Verkneten der Baumwoll- oder Viskosefasern mit der Viskoselösung und anschließend auch mit dem Glaubersalz, Ausstreichen der aus dem Kneter erhaltenen Masse auf ein Transportband, Koagulieren unter Hitzeeinwirkung, Waschen und Trocknen. Das Koagulationsbad enthält allgemein Natriumhydroxid und ist damit stark alkalisch (pH ≥ 13).

## Patentansprüche

1. Schwammtuch auf der Basis von regenerierter Cellulose, das mit einer Innenverstärkung versehen ist, **dadurch gekennzeichnet, daß** die Fällung und Regenerierung in einem aus Glaubersalz, NaOH und Wasser bestehenden Bad mit einem pH-Wert von 13 oder mehr erfolgt und daß die Innenverstärkung aus Viskosefasem mit einer Stapelfaserlänge von 5 bis 50 mm besteht und daß es mit einem biocid wirkenden Mittel imprägniert ist, das nach der Regeneration aufgebracht wird.

2. Schwammtuch gemäß Anspruch 1, **dadurch gekennzeichnet, daß** das biocid wirkende Mittel ein Isothiazolon-, Benzisothiazolon- oder Benzimidazol-Derivat ist.

3. Schwammtuch gemäß Anspruch 1, **dadurch gekennzeichnet, daß** das biocid wirkende Mittel ein quatemäres Ammoniumsalz ist.

4. Schwammtuch gemäß Anspruch 1, **dadurch gekennzeichnet, daß** das biocid wirkende Mittel Glycerin oder Propan-1,2-diol ist.

5. Schwammtuch gemäß Anspruch 1, **dadurch gekennzeichnet, daß** die Stapelfaserlänge der Viskosefasem 10 bis 30 mm beträgt.

6. Schwammtuch gemäß Anspruch 1, **dadurch gekennzeichnet, daß** der Anteil der Innenverstärkung 5 bis 50 Gew.-% beträgt, bezogen auf das Trockengewicht des Schwammtuches.

7. Schwammtuch gemäß Anspruch 1, **dadurch gekennzeichnet, daß** es mit einer wäßrigen Magnesiumchloridlösung befeuchtet ist.

8. Schwammtuch auf der Basis von regenerierter Cellulose, das mit einer Innenverstärkung versehen ist, **dadurch gekennzeichnet, daß** die Fällung und Regenerierung in einem aus Glaubersalz, NaOH und Wasser bestehenden Bad mit einem pH-Wert von 13 oder mehr erfolgt und daß die Innenverstärkung aus Baumwollfasem mit einer Stapelfaserlänge von 5 bis 50 mm besteht und daß es mit einem baktericid wirkenden Mittel imprägniert ist, das nach der Regeneration aufgebracht wird.

9. Schwammtuch gemäß Anspruch 8, **dadurch gekennzeichnet, daß** das baktericid wirkende Mittel ein Isothiazolon-, Benzisothiazolon- oder Benzimidazol-Derivat ist.

10. Schwammtuch gemäß Anspruch 8, **dadurch gekennzeichnet, daß** die Stapelfaserlänge der Baumwollfasem 10 bis 30 mm beträgt.

11. Schwammtuch gemäß Anspruch 8, **dadurch gekennzeichnet, daß** das bactericid wirkende Mittel ein Penicillin, Cephalosporin, Tetracyclin, ein Pyridiniumsalz oder eine Biguanidverbindung ist.

12. Schwammtuch gemäß Anspruch 8, **dadurch gekennzeichnet, daß** der Anteil der Innenverstärkung 5 bis 50 Gew.-% beträgt, bezogen auf das Trockengewicht des Schwammtuches.

13. Schwammtuch gemäß Anspruch 8, **dadurch gekennzeichnet, daß** es mit einer wäßrigen Magnesiumchlorid-Lösung befeuchtet ist.

## Claims

1. A sponge cloth which is based on regenerated cellulose and has been provided with an internal reinforcement wherein said sponge cloth being obtained by coagulating and regenerating the cellulose in a bath which has a pH of 13 or higher and which comprises Glauber's salt, NaOH and water, and the internal reinforcement consists of viscose fibers having a staple fiber length of 5 to 50 mm, wherein the sponge cloth is impregnated with a biocidally active agent which is applied after regeneration.

2. The sponge cloth of claim 1, wherein the biocidally active agent is an isothiazolone, benzisothiazolone or benzimidazole derivative.

3. The sponge cloth of claim 1, wherein the biocidally active agent is a quaternary ammonium salt.

4. The sponge cloth of claim 1, wherein the biocidally active agent is glycerol or 1,2-propanediol.

5. The sponge cloth of claim 1, wherein the staple fiber length of the viscose fibers is 10 to 30 mm.

6. The sponge cloth of claim 1, wherein the proportion of the dry weight of the sponge cloth attributable to the internal reinforcement is 5 to 50% by weight.

7. The sponge cloth of claim 1, moistened with an aqueous magnesium chloride solution.

8. The sponge cloth which is based on regenerated cellulose and has been provided with an internal reinforcement wherein said sponge cloth being obtained by coagulating and regenerating the cellulose in a bath which has a pH of 13 or higher and which comprises Glauber's salt, NaOH and water, and the internal reinforcement consists of cotton fibers having a staple fiber length of 5 to 50 mm, wherein the sponge cloth is impregnated with a bactericidally active agent which is applied after regeneration.

9. The sponge cloth of claim 8, wherein the bactericidally active agent is an isothiazolone, benzisothiazolone or benzimidazole derivative.

10. The sponge cloth of claim 8, wherein the staple fiber length of the cotton fibers is 10 to 30 mm.

11. The sponge cloth of claim 8, wherein the bactericidally active agent is a penicillin, cephalosporin, tetracyclin, a pyridinium salt or a biguanide compound.

12. The sponge cloth of claim 8, wherein the proportion of the dry weight of the sponge cloth attributable to the internal reinforcement is 5 to 50% by weight.

13. The sponge cloth of claim 8, moistened with an aqueous magnesium chloride solution.

## Revendications

1. Tissu éponge à base de cellulose régénérée qui est pourvu d'un renforcement intérieur, **caractérisé en ce que** la précipitation et la régénération se font dans un bain comprenant un sel de Glauber, du NaOH et de l'eau à une valeur de pH de 13 ou plus, et **en ce que** le renforcement intérieur est constitué de fibres de viscose avec une longueur de fibre comprise entre 5 et 50 mm, et **en ce qu'**il est imprégné avec un agent à action biocide qui est appliqué après la régénération.

2. Tissu éponge selon la revendication 1, **caractérisé en ce que** l'agent à action biocide est un dérivé d'isothiazolone, de benzisothiazolone ou de benzimidazole.

3. Tissu éponge selon la revendication 1, **caractérisé en ce que** l'agent à action biocide est un sel d'ammonium quaternaire.

4. Tissu éponge selon la revendication 1, **caractérisé en ce que** l'agent à action biocide est de la glycérine ou du propanediol-1,2.

5. Tissu éponge selon la revendication 1, **caractérisé en ce que** la longueur des fibres de viscose est comprise entre 10 et 30 mm.

6. Tissu éponge selon la revendication 1, **caractérisé en ce que** la portion de renforcement intérieur est comprise entre 5 et 50% en poids par rapport au poids à l'état sec du tissu éponge.

7. Tissu éponge selon la revendication 1, **caractérisé en ce qu'**il est humidifié avec une solution aqueuse de chlorure de magnésium.

8. Tissu éponge à base de cellulose régénérée qui est pourvu d'un renforcement intérieur, **caractérisé en ce que** la précipitation et la régénération se font dans un bain comprenant un sel de Glauber, du NaOH et de l'eau à une valeur pH de 13 ou plus, et **en ce que** le renforcement intérieur est constitué de fibres de viscose avec une longueur de fibre comprise entre 5 et 50 mm, et **en ce qu'**il est imprégné avec un agent à action bactéricide qui est appliqué après la régénération.

9. Tissu éponge selon la revendication 8, **caractérisé en ce que** l'agent à action bactéricide est un dérivé d'isothiazolon, de benzisothiazolon ou de benzimidazole.

10. Tissu éponge selon la revendication 8, **caractérisé en ce que** la longueur des fibres de viscose est comprise entre 10 et 30 mm.

11. Tissu éponge selon la revendication 8, **caractérisé en ce que** l'agent à action bactéricide est une pénicilline, une céphalosporine, une tétracycline ou un sel de pyridium ou un composé de biguanide.

12. Tissu éponge selon la revendication 8, **caractérisé en ce que** la portion de renforcement intérieur est comprise entre 5 et 50% en poids par rapport au poids sec du tissu éponge.

13. Tissu éponge selon la revendication 8, **caractérisé en ce qu'**il est humidifié avec une solution aqueuse de chlorure de magnésium.
